# EUROPEAN PATENT APPLICATION

(11) **EP 3 632 494 A1**
(43) Date of publication of application: **08.04.2020**
(21) Application number: 18806213.7
(22) Date of filing: 17.05.2018
(51) Int. Cl.: A61M 25/00

(54) **CATHETER**

(30) Priority: 26.05.2017 JP 2017104985; 26.05.2017 JP 2017104986
(71) Applicant: Sumitomo Bakelite Co., Ltd., Shinagawa-ku Tokyo 140-0002 (JP)
(72) Inventor: ONOZUKA Iji, Akita-shi Akita 011-8510 (JP)
(74) Representative: Ter Meer Steinmeister & Partner
(86) International application number: PCT/JP2018/019113
(87) International publication number: WO 2018/216596

(57) **Abstract**

The present invention provides a catheter provided with a tubular main body. The catheter is characterized in that: the tubular main body has defined therein a first length region and a second length region which is adjacent to the first length region and which is located closer to the distal side than the first length region; the resin hardness of the second length region is lower than the resin hardness of the first length region; and the distance from a plane perpendicular to the axial direction of the tubular main body to the joining line between the first length region and the second length region is non-uniform.

## Description

### Technical Field

The present invention relates to a catheter.

### Background Art

In a catheter, it is desirable to have softness for causing an excess load not to be applied to body tissue when the catheter is inserted into a body cavity such as a blood vessel, and at the same time, to have "stiffness" suitable to obtain good runnability, blood vessel selectivity, or torque properties of the catheter. In this specification, the stiffness refers to the characteristic of a portion (a main body) of the catheter, which is inserted into the body cavity, the characteristic being determined by an elastic force, a hardness, flexibility, and the like. If the stiffness of the catheter is suitable, the catheter can operate in the body cavity following an operator's operation.

For example, PTL 1 discloses a catheter in which the hardness of an outer layer of the catheter is reduced in stages from a proximal end portion toward a distal end portion of the catheter. More specifically, the catheter disclosed in PTL 1 is a tube having a main body which includes an inner layer, an outer layer, and an intermediate layer provided between the inner layer and the outer layer. The tube has a tapered portion in which a diameter thereof continuously increases from a tip toward a base end. PTL 1 discloses a catheter in which the tapered portion of the catheter main body is made such that the hardness of resin that is the material thereof is reduced in stages toward a distal end portion and the diameter of the tapered portion is reduced toward the distal end portion, and whereby the rigidity of the catheter main body decreases as it moves towards the distal end portion.

### Citation List

### Patent Literature

[PTL 1] Japanese Unexamined Patent Application, First Publication No. 2012-196389

### Summary of Invention

### Technical Problem

Incidentally, in a case where the rigidity of the catheter main body is changed along an axial direction of the tube, materials having different hardness are switched in the axial direction, or the rigidity resulting from the tubular form of the tube is changed.

The rigidity of the catheter main body changes according to the material switching position or the tubular form of the tube. However, variation in the material switching position appears as variation in the rigidity of the catheter and eventually a function such as blood vessel selectivity, runnability in a blood vessel, or torque properties. For this reason, in order to control the function of the catheter main body, it is desirable to suppress variation in the material switching position for each catheter.

Further, if the hardness steeply changes at the position where the material is switched, there is a possibility that a phenomenon in which the catheter main body is folded at the switching position (hereinafter also referred to as "kink") may occur. For this reason, in the field where the catheter is handled, it is desirable to increase kink resistance of the catheter.

However, PTL 1 described above does not disclose any configuration for suppressing variation in material switching position for each product.

PTL 1 described above does not disclose any configuration for increasing the kink resistance of the catheter main body.

The present invention has been made in view of the above points and has an object to provide a catheter which has excellent manufacturing stability by suppressing variation in switching position while switching the hardness of a material in an axial direction, and eventually has small variation in a function such as blood vessel selectivity, runnability in a blood vessel, or torque properties.

Further, the present invention has been made in view of the above points and has an object to provide a catheter in which it is possible to prevent the occurrence of kink resulting from discontinuity of hardness while switching the hardness of a material in an axial direction.

### Solution to Problem

According to the present invention, a catheter is provided, including: a tubular main body made of resin and having a change portion in which rigidity resulting from a tubular form continuously decreases from a base end side toward a tip side in an axial direction, in which a first length region and a second length region located closer to a distal side than the first length region are defined in the tubular main body further on the distal side than the change portion, and a resin hardness of the second length region is lower than a resin hardness of the first length region.

Further, according to the present invention, a catheter is provided, including: a tubular main body, in which a first length region and a second length region adjacent to the first length region and located closer to a distal side than the first length region are defined in the tubular main body, a resin hardness of the second length region is lower than a resin hardness of the first length region, and a joining line between the first length region and the second length region is non-uniform in distance from a plane perpendicular to an axial direction of the tubular main body.

### Advantageous Effects of Invention

According to the present invention, it is possible to provide a catheter that has excellent manufacturing stability by suppressing variation in switching position while switching rigidity in an axial direction and eventually has small variation in a function such as blood vessel selectivity, runnability in a blood vessel, or torque properties.

Further, according to the present invention, it is possible to provide a catheter in which it is possible to prevent the occurrence of kink due to discontinuity of rigidity while switching the rigidity in an axial direction.

### Brief Description of Drawings

FIG. 1 is a diagram describing a catheter of a first embodiment and a second embodiment of the present invention.
FIG. 2 is a cross-sectional view of FIG. 1.
FIG. 3 is a longitudinal sectional view of FIG. 2.
FIG. 4 is a schematic longitudinal sectional view showing a predetermined range including the tip of a tubular main body shown in FIG. 1, in an enlarged manner.
FIG. 5 is a diagram describing a position of a joining line shown in FIG. 4.
FIG. 6 is a diagram describing the shape of the joining line shown in FIGS. 4 and 5.
FIG. 7 is a diagram describing modification examples of the first embodiment and the second embodiment of the present invention.
FIG. 8 is a schematic longitudinal sectional view showing a predetermined range including a change portion of the tubular main body shown in FIG. 1, in an enlarged manner.
FIG. 9 is a perspective view showing a portion of the change portion of the tubular main body shown in FIG. 8 and is a diagram showing a joining line in the change portion.
FIG. 10 is a diagram describing a circumferential direction of a tubular main body and a position of a joining line in a catheter of the second embodiment of the present invention.

### Description of Embodiments

Hereinafter, embodiments of the present invention will be described based on the drawings. In all the drawings, the same constituent elements are denoted by the same reference numerals, and detailed descriptions thereof are appropriately omitted so as not to overlap.

### [First Embodiment]

### (Catheter)

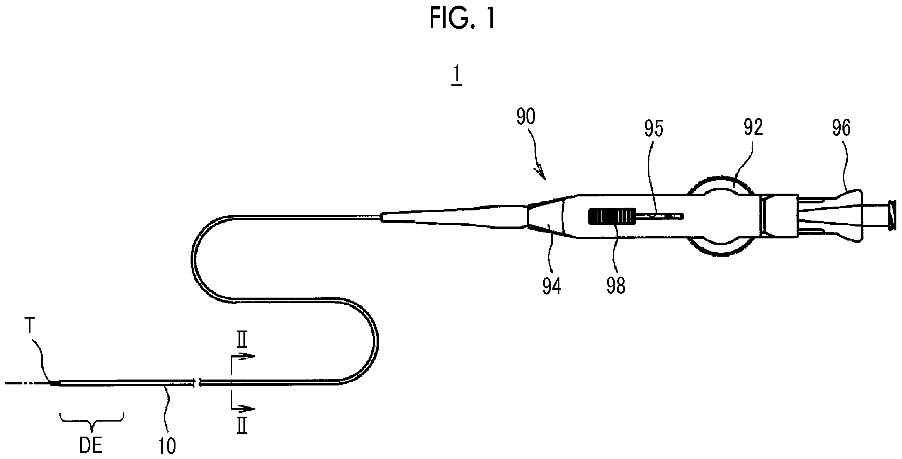

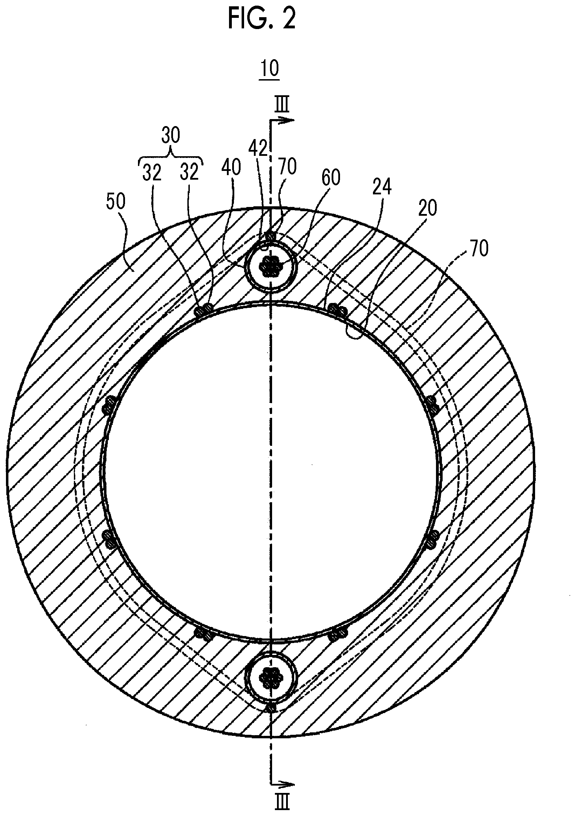

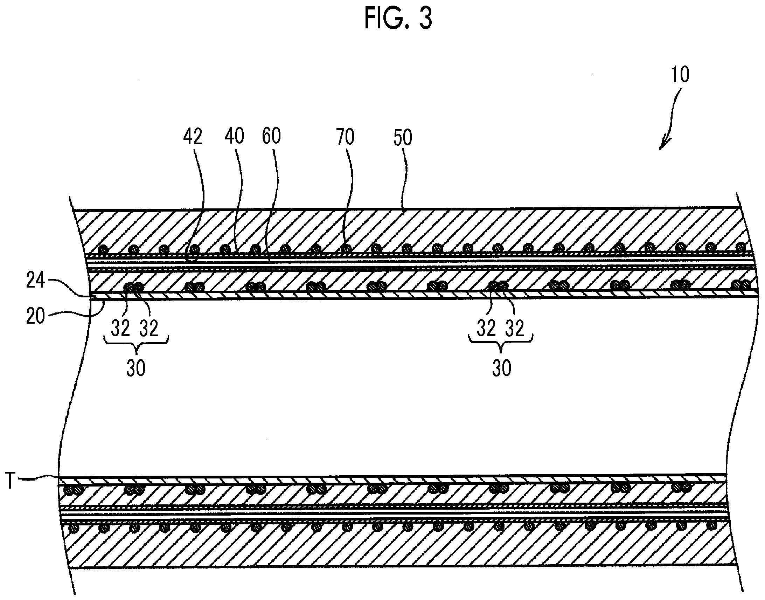

FIG. 1 is a diagram describing a catheter 1 of a first embodiment of the present invention. FIG. 2 is a cross-sectional view of the catheter 1 taken along arrow II-II shown in FIG. 1. FIG. 3 is a longitudinal sectional view of the catheter 1 taken along arrow III-III shown in FIG. 2. The catheter 1 of this embodiment is an active catheter in which an operating line 60 is disposed in the interior of a tubular main body 10 and a tip T of the tubular main body 10 is bent by operating an operation part 90 and a bending operation part 92. However, the catheter according to the present invention is not limited thereto and may be an inactive catheter which is not provided with the operating line 60 or the operation part 90.

The catheter 1 of this embodiment has the operation part 90 provided at a base end of the tubular main body 10. Further, an end portion on the side opposite to the side where the tubular main body 10 is connected to the operation part 90 is defined as the tip T. The operation part 90 configures an operation mechanism for performing a bending operation of a distal portion DE of the tubular main body 10 together with the operating line 60 (refer to FIGS. 2 and 3). The operation part 90 of this embodiment has a main body case 94 which is gripped by an operator's hand, and the bending operation part 92 which is provided to be rotatable with respect to the main body case 94. The base end of the tubular main body 10 is introduced into the interior of the main body case 94. A recessed portion 95 is formed in the main body case 94 at a position which is in contact with the bending operation part 92. A slider 98 which slides to be able to advance toward and retreat from the bending operation part 92 is provided in the recessed portion 95.

The operation part 90 is provided with a hub 96 provided in communication with a main lumen 20 of the tubular main body 10. A syringe (not shown) is mounted to the hub 96. The hub 96 is provided at a rear end portion of the main body case 94, and the syringe is mounted from the rear of the hub 96. A drug solution or the like is injected into the hub 96 by the syringe, whereby the drug solution or the like can be supplied into the body cavity of a subject through the main lumen 20 (FIGS. 2 and 3) which is a hollow portion of the tubular main body 10. As the drug solution or the like, in addition to a drug solution such as a contrast agent, a liquid anticancer agent, a physiological saline, or NBCA (n-butyl-2-cianoacrylate) which is used as an instant adhesive, a medical device such as a detachable coil or beads (embolus globular substances) can be given as an example.

The tubular main body 10 shown in FIG. 1 is also called a sheath and is a hollow tubular and long member in which the main lumen 20 is formed in the interior thereof. More specifically, the tubular main body 10 is formed to have an outer diameter which allows the tubular main body 10 to enter into a blood vessel. The tubular main body 10 is a tubular main body made of resin and having a "change portion" in which rigidity resulting from a tubular form continuously decreases from the base end side toward the tip side in an axial direction (a direction of an axis passing through the center point of the cross section of the main lumen 20). Here, the "rigidity resulting from a tubular form" is rigidity resulting from the inner diameter or outer diameter of the tubular main body 10, and eventually the wall thickness of the tubular main body 10. That is, the "tubular form" includes at least one of the wall thickness, the outer diameter, and the inner diameter of the tubular main body as an element. The rigidity resulting from the form of the tubular main body 10 becomes small if the inner diameter or the outer diameter of the tubular main body 10 is relatively small, and becomes large if the inner diameter or the outer diameter of the tubular main body 10 is large. Further, the rigidity becomes large if the wall thickness of the tubular main body 10 is relatively thick, and becomes small if the wall thickness of the tubular main body 10 is thin.

In order to change the rigidity resulting from the tubular form of the tubular main body 10 in a "continuous" manner, in an example of this embodiment, the tubular main body 10 has a tapered shape in which the outer diameter of the tubular main body 10 continuously increases (the wall thickness becomes thick) without changing the outer diameter of the main lumen 20 from the tip toward the base end. In this way, the rigidity resulting from the form of the tubular main body 10 continuously increases from the tip T toward the base end.

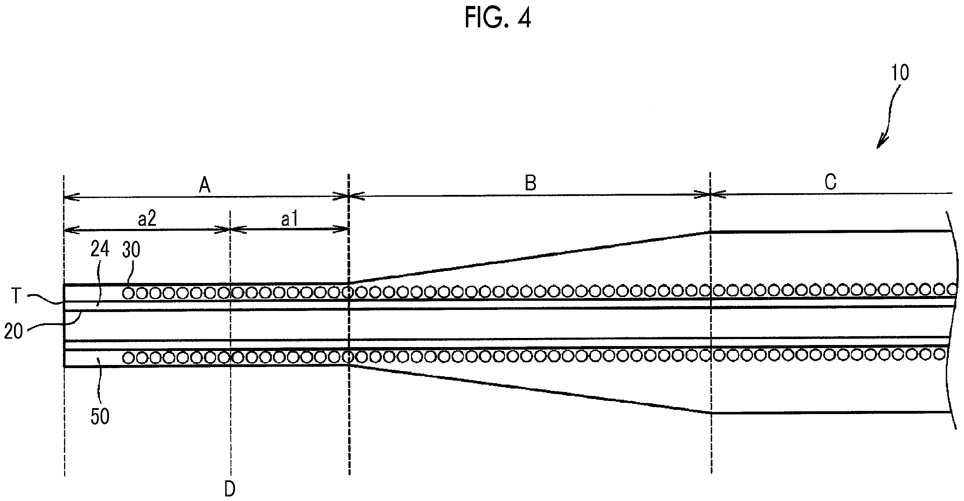

Further, in the tubular main body 10, a first length region a1 (refer to FIG. 4) and a second length region a2 (refer to FIG. 4) located closer to the distal side than the first length region are defined in the tubular main body further on the distal side than the change portion. In this embodiment, the resin hardness of the second length region is lower than the resin hardness of the first length region. That is, in this embodiment, in the tubular main body 10, a predetermined length range further on the distal side (the tip T side) than the tapered portion is defined as the first length region. Further, a predetermined length range located closer to the distal side than the first length region is defined as the second length region. Then, the material of the first length region and the material of the second length region are switched such that the hardness of the material of the second length region is lower than the hardness of the material of the first length region.

The first length region a1 and the second length region a2 will be described in detail later.

Here, prior to the description of the first length region and the second length region, an example of the structure of the tubular main body 10 will be described. However, the tubular main body 10 according to the present invention is not limited to the following laminated configuration.

As shown in FIGS. 2 and 3, the tubular main body 10 has a laminated structure. The tubular main body 10 is configured by laminating an inner layer (a main tube) 24 and an outer layer 50 in order from the inner diameter side with the main lumen 20 as the center. A hydrophilic layer (not shown) is formed on the outer surface of the outer layer 50. The inner layer 24 and the outer layer 50 are formed of a flexible resin material and each has a circular tube shape and a substantially uniform thickness in the circumferential direction.

The inner layer 24 is the innermost layer of the tubular main body 10, and the main lumen 20 is defined by the inner wall surface thereof. The shape of the cross section of the main lumen 20 is not particularly limited. However, in this embodiment, the shape of the cross section of the main lumen 20 is a circular shape. In this specification, the term "cross section of the main lumen" refers to the cross section of a shape defined by the outer peripheral surface of the main lumen 20 (a cylinder long in the axial direction of the main lumen 20). Further, in this embodiment, the diameter of the cross section of the tubular main body 10 is a circular shape, and the diameter of the tubular main body 10 differs depending on the position in a longitudinal direction (the axial direction of the main lumen 20), so that in a part or the entire length region of the tubular main body 10, the tubular main body 10 has a tapered shape in which the diameter of the tubular main body 10 continuously increases from the tip toward the base end.

As the material of the inner layer 24, a fluorine-based thermoplastic polymer resin can be given as an example. As the fluorine-based thermoplastic polymer resin, specifically, polytetrafluoroethylene (PTFE), polyvinylidene fluoride (PVDF), and perfluoroalkoxy fluororesin (PFA) can be given as examples. The inner layer 24 is configured with such a fluorine-based polymer material, whereby the delivery property when supplying the drug solution or the like through the main lumen 20 is improved. Further, the sliding resistance when inserting a guide wire or the like into the main lumen 20 is reduced.

A wire reinforcement layer 30 is a protective layer that is provided further on the inner diameter side than the operating line 60 in the tubular main body 10 to protect the inner layer 24. The wire reinforcement layer 30 is present on the inner diameter side of the operating line 60, whereby it is possible to prevent the operating line 60 from penetrating from the outer layer 50 to the inner layer 24 and being exposed to the main lumen 20.

The wire reinforcement layer 30 is formed by winding a reinforcement wire 32. As the material of the reinforcement wire 32, in addition to a metal material such as tungsten (W), stainless steel (SUS), a nickel titanium alloy, steel, titanium, copper, a titanium alloy, or a copper alloy, a resin material such as polyimide (PI), polyamideimide (PAI), or polyethylene terephthalate (PET), which has higher shear strength than the inner layer 24 and the outer layer 50, can be used. In this embodiment, a fine stainless steel wire is used as the reinforcement wire 32.

The number of the reinforcement wires 32 or the number of meshes is not particularly limited. Here, the number of meshes of the wire reinforcement layer 30 refers to the number of intersections (the number of eyes) per unit length (1 inch) viewed in the extending direction of the reinforcement wire.

The reinforcement wire 32 is wound obliquely around the inner layer 24. An angle formed by the extending direction of the reinforcement wire 32 with respect to the circumferential direction around the inner layer 24 is referred to as a pitch angle of the reinforcement wire 32. In a case where the reinforcement wire 32 is wound at a dense pitch, the pitch angle becomes a small angle. Conversely, in a case where the reinforcement wire 32 is wound at a shallow angle along the axis of the tubular main body 10, the pitch angle becomes a large angle close to 90 degrees. The pitch angle of the reinforcement wire 32 of this embodiment is not particularly limited. However, it can be 30 degrees or more, preferably 45 degrees or more and 75 degrees or less.

The number of the reinforcement wires 32 configuring the wire reinforcement layer 30 is not particularly limited. However, in this embodiment, the wire reinforcement layer 30 formed by 16 reinforcement wires 32 is illustrated.

A sub-tube 40 is a hollow tubular member that defines a sub-lumen. The sub-tube 40 is embedded in the outer layer 50. The sub-tube 40 can be made of, for example, a thermoplastic polymer material. As the thermoplastic polymer material, a low friction resin material such as polytetrafluoroethylene (PTFE), polyetheretherketone (PEEK), or a tetrafluoroethylene/hexafluoropropylene copolymer (FEP) can be given as an example. The sub-tube 40 is made of a material having higher bending rigidity and tensile elastic modulus than the outer layer 50.

Two sub-tubes 40 are disposed on the same circumference so as to surround the main lumen 20. Instead of this embodiment, three or four sub-tubes 40 may be disposed around the main lumen 20 at equal intervals. In this case, the operating lines 60 may be disposed in all the sub-tubes 40, or the operating lines 60 may be disposed in some of the sub-tubes 40. A sub-lumen 42 is formed in the sub-tube 40, and the operating line 60 is movably inserted into the sub-lumen 42.

As described above, the catheter 1 of this embodiment is an active type, and is provided with the sub-tube 40 and a holding wire 70, in addition to the operating line 60 and the operation part 90.

However, in a case where the catheter of this embodiment is an inactive type, such a configuration is not necessary.

The operating line 60 is loosely inserted to be slidable with respect to the sub-tube 40. The tip of the operating line 60 is fixed to the distal portion DE of the tubular main body 10. A tensile force is applied to a position eccentric with respect to the axis of the tubular main body 10 by pulling the operating line 60 to the base end side, and therefore, the tubular main body 10 is bent. For this reason, it is possible to cause the catheter 1 to select and enter any blood vessel among the branched blood vessels.

The operating line 60 may be formed of a single wire. However, it may be a stranded wire formed by twisting a plurality of thin wires together. The number of fine wires configuring one stranded wire of the operating line 60 is not particularly limited but is preferably 3 or more.

As the operating line 60, a metal wire such as low-carbon steel (a piano wire), stainless steel (SUS), a corrosion-resistant coated steel wire, titanium or a titanium alloy, or tungsten can be used. In addition to this, as the operating line 60, polyvinylidene fluoride (PVDF), high-density polyethylene (HDPE), poly(paraphenylenebenzobisoxazole) (PBO), polyetheretherketone (PEEK), polyphenylene sulfide (PPS), polybutylene terephthalate (PBT), polyimide (PI), polytetrafluoroethylene (PTFE), or a polymer fiber such as a boron fiber can be used.

The holding wire 70 is wound in a spiral shape so as to surround the outside of the pair of sub-tubes 40 disposed to face each other around the main lumen 20. The winding shape of the holding wire 70 of this embodiment is an approximately elliptical shape or an approximately rhombic shape, in which the direction of the major axis thereof corresponds to the arrangement direction of the sub-tubes 40. In FIG. 3, the holding wire 70 in which the winding shape forms an approximately rhombic shape is shown by a broken line. The holding wire 70 is in contact with the peripheral surfaces of the sub-tubes 40, specifically, the outer surface corresponding to the side opposite to the axis of the main lumen 20. Here, the approximately rhombic shape means that a first diagonal line is longer than a second diagonal line and the first diagonal line and the second diagonal line are substantially orthogonal to each other. The approximately rhombic shape mentioned here includes a flat polygon such as a kite shape, a flat hexagon, or a flat octagon, in addition to a rhombus. Further, the approximately elliptical shape includes an eccentric circle shape such as an ovoid shape, in addition to an elliptical shape or an oval shape.

In this embodiment, an aspect is exemplified in which the shape of the cross section of the main lumen 20 is a circular shape, the main lumen 20 is disposed at the center of the tubular main body 10, and the two sub-tubes 40 are disposed around the main lumen 20 so as to face each other by 180 degrees. However, the shape of the cross section of the main lumen 20 is not limited to a circular shape and may be another shape such as an ellipse or a rectangle. Further, three or more (N) sub-tubes 40 may be equally distributed and disposed around the main lumen 20. In this case, the winding shape of the holding wire 70 may be a corner-rounded N-polygonal shape with each sub-tube 40 as a comer.

As the material of the holding wire 70, any of the above-described metal materials or resin materials that can be used as the reinforcement wire 32 can be used. In this embodiment, the holding wire 70 is formed to include a material of a different type from that of the reinforcement wire 32. The ductility of the holding wire 70 is preferably higher than the ductility of the reinforcement wire 32. Specifically, austenitic soft stainless steel (W1 or W2), which is an annealed material, or copper or a copper alloy is used for the holding wire 70, while tungsten or stainless spring steel can be used for the reinforcement wire 32.

The outer layer 50 is a circular tube-shaped portion that configures the main wall thickness of the tubular main body 10. The wire reinforcement layer 30, the sub-tube 40, and the holding wire 70 are provided in order from the inner diameter side in the interior of the outer layer 50. The wire reinforcement layer 30 and the holding wire 70 are disposed coaxially with the tubular main body 10.

In this embodiment, the resin hardness of the first length region and the resin hardness of the second length region of the tubular main body 10 being different from each other means that the resin hardness of the outer layer 50 configuring the main wall thickness of the tubular main body 10 is different. However, the outer layer 50 may be a single layer or a multilayer, and when the outer layer 50 is a multilayer, it indicates that the resin hardness of the first length region and the resin hardness of the second length region are different from each other with respect to the thickest layer of a plurality of layers.

As the material of the outer layer 50, a thermoplastic polymer material can be used. As the thermoplastic polymer material, polyimide (PI), polyamideimide (PAI), polyethylene terephthalate (PET), polyethylene (PE), polyamide (PA), polyamide elastomer (PAE), nylon elastomer such as polyether block amide (PEBA), polyurethane (PU), ethylene-vinyl acetate resin (EVA), polyvinyl chloride (PVC), or polypropylene (PP) can be given as an example.

An inorganic filler may be mixed with the outer layer 50. As the inorganic filler, a contrast agent such as barium sulfate or bismuth subcarbonate can be exemplified. The contrast agent is mixed with the outer layer 50, whereby the X-ray contrast property of the tubular main body 10 in the body cavity can be improved.

In a case of switching the resin hardness by making the resin material of the first length region and the resin material of the second length region different from each other, it is preferable in terms of adhesion that the resin material of the first length region and the resin material of the second length region are the same type of resin materials having different hardness. The same type of resin materials having different hardness refer to, for example, resin materials in which there is a commonality in resin structure and the degree of cross-link is different, or the hardness is different resulting from a different component or addition amount of a filler or an elastomer, or the like.

Next, the change portion, the first length region, and the second length region of this embodiment described above will be described.

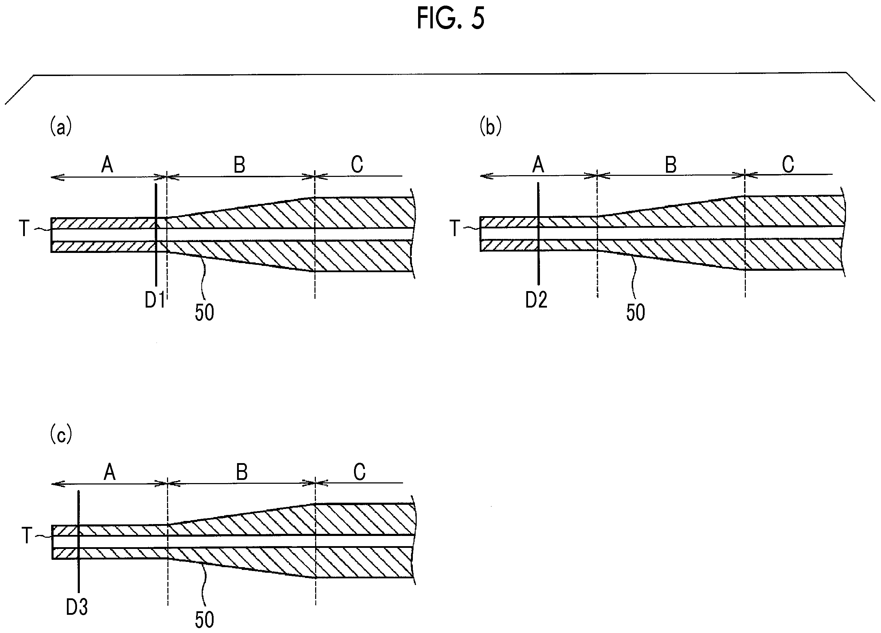

FIG. 4 is a schematic longitudinal sectional view showing the distal portion DE of the tubular main body 10 in an enlarged manner. The illustrated distal portion DE has a tapered change portion B in which the outer diameter of the tubular main body 10 continuously decreases from the base end side toward the tip side, a proximal region C located closer to the proximal side than the change portion B. and a distal region A located closer to the distal side than the change portion B. The inner circumference of the main lumen 20 is constant even in the change portion B. The inclination of the outer diameter of the tubular main body 10 may be the same throughout the change portion B, or the inclination may change in the middle. The outer diameter of the main lumen 20 may be constant even in the change portion B, or may have the same inclination throughout the change portion B, or the inclination may change in the middle. Further, the distal region A includes the first length region a1 and the second length region a2 further on the distal side than the first length region a1. In FIG. 4, the position of a joining line D, which is a boundary between the first length region a1 and the second length region a2, that is, a boundary line in which resins that are different in hardness are joined to each other, is shown. Further, in FIG. 5, the joining lines are shown with joining lines D1, D2, and D3. In FIGS. 4 and 5, the position of the joining line is shown with a straight line. However, as will be described later, in this embodiment, the joining line D can be a curved line.

From the viewpoint of a balance that has excellent blood vessel selectivity or guide wire followability in a blood vessel branch while providing good runnability in a blood vessel or torque properties of the catheter, it is preferable that, with regard to the resin hardness of the first length region a1 and the resin hardness of the second length region a2, the value of (Shore hardness of the first length region a1)/(Shore hardness of the second length region a2) be set to 1.1 or more and 2.0 or less. Further, from the viewpoint of a balance that has excellent blood vessel selectivity or guide wire followability in a blood vessel branch while providing good runnability in a blood vessel or torque properties of the further catheter, it is preferable that the value of (Shore hardness of the change portion B)/(Shore hardness of the second length region a2) be set to 1.1 or more and 2.0 or less.

Among these, in the example shown in FIG. 4, nylon elastomer is used for the outer layers 50 in the distal region A, the change portion B, and the proximal region C, and the length, the outer diameter φ of the outer layer 50 (an index of the area of the cross section), and the Shore hardness of the outer layer 50 can be set as follows, for example.
Distal region A: length 15 mm, φ0.8 mm, Shore hardness 25D (the second length region a2), Shore hardness 35D (the first length region a1)
Change portion B: length 3 mm, φ0.9 mm to 0.8 mm, Shore hardness 35D
Proximal region C: length 10 mm, φ0.9 mm, Shore hardness 35D

That is, in the above configuration, the resin hardness of the outer layer 50 configuring each of the change portion B and the proximal region C is uniform.

However, the size or the resin hardness of each of the distal region A, the change portion B, and the proximal region C is not limited to the above-described numerical values or ratios as a matter of course. The size and resin hardness of each part are determined according to the use or the like of the catheter 1.

FIGS. 5(a) to 5(c) are diagrams showing a plurality of examples of the joining line between the first length region a1 and the second length region a2 in the tubular main body 10 on the distal side. FIG. 5(a) shows an example in which the joining line D1 is located close to the change portion B in the distal region A. The minimum distance between the joining line D1 and the end portion (distal end) closest to the distal side of the change portion B can be set to be, for example, about the wall thickness in the joining line D1 of the tubular main body 10, and more preferably, it may be longer than half (radius) of the outer diameter of the tubular main body 10 at the boundary between the distal region A and the change portion B.

FIG. 5(b) shows the joining line D2 which is at a position closer to the tip T than the joining line D1. Further, FIG. 5(c) shows the joining line D3 which is at a position closer to the tip T than the joining line D2. In this manner, the joining line may be anywhere in the distal region A. The closer the joining line D is located to the tip T, the stronger the stiffness in the vicinity of the tip of the tubular main body 10 becomes, and the stronger the characteristic such as the runnability in a blood vessel or the torque properties of the catheter becomes. Further, conversely, the farther the joining line D is located from the tip T, the weaker the stiffness in the vicinity of the tip of the tubular main body 10 becomes, and the further the blood vessel selectivity or guide wire followability in a blood vessel branch of the catheter is improved. The position of the joining line D is determined according to the use of the catheter 1, the hardness of the resin material, or the like.

The switching of the resin material in the distal region A is not limited to one location and may be performed in multiple stages at a plurality of locations. Further, the first length region a1 and the second length region a2 are not limited to those adjacent to each other, and may be disposed with another region interposed therebetween.

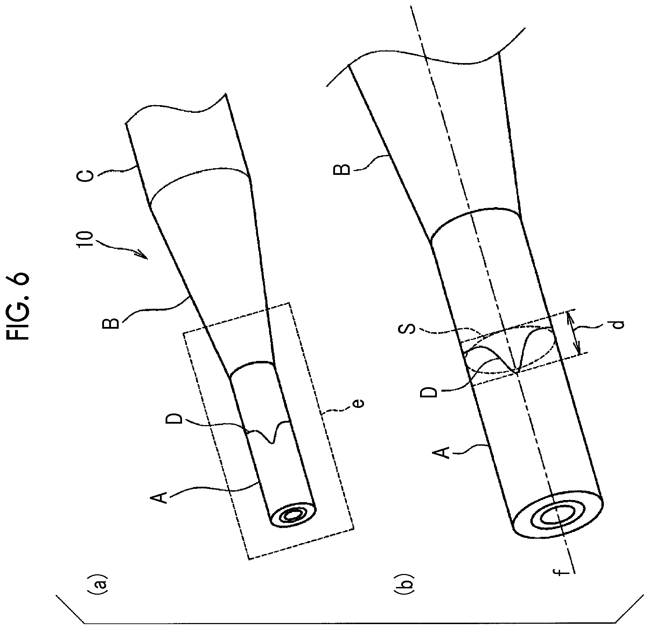

FIGS. 6(a) and 6(b) are perspective views showing the portion of the distal region A in the tubular main body 10 and are diagrams showing the joining line D in the distal region A. FIG. 6(a) shows the entire distal region A, and FIG. 6(b) is an enlarged diagram of the portion indicated by a broken line e in FIG. 6(a). A distance d of the joining line D on the distal region A from a plane (a cross section S of the tubular main body 10) perpendicular to the direction of an axis f of the tubular main body 10 is non-uniform. Here, the distance d from the cross section S refers to the distance d between a point on the circumference of the cross section S and the intersection of a line extending parallel to the axis f from this point and the joining line D. and the cross section S is in contact with the point closest to the base end side of the joining line D (d=0). As shown in FIG. 6(b), the distance d varies depending on the position of the joining line D. In this embodiment, the distal region A is designed such that the concavity and convexity of the joining line D are provided in one cycle to five cycles. That is, the joining line D has a shape in which 1 to 5 convex portions protrude toward the tip T side or the base end side. Further, it is more preferable that the concavity and convexity of the joining line D be provided in two cycles to five cycles. In this way, the hardness gradually changes in the joining line D, and thus, when the catheter 1 is bent, the region ranging from the first length region a1 to the second length region a2 is smoothly curved, and the blood vessel selectivity or guide wire followability in a blood vessel branch is improved. Further, it is possible to prevent cracks or damage from occurring in the boundary between the first length region a1 and the second length region a2. Further, even if slight variation occurs in the position of the joining line D, it is possible to absorb variation in a function such as the runnability in a blood vessel or the torque properties for each product of the catheter 1.

It is favorable if the joining line D is a curved line surrounding the periphery of the distal region A of the tubular main body 10, and the joining line D is not limited to a curved line in which the cycle of the concavity and convexity is substantially constant. Further, the amplitude of the concavity and convexity of the joining line D is also not limited to being constant.

In this embodiment, as described above, by thinning the tubular main body 10 from the base end toward the tip T, the morphological hardness of the tubular main body 10 is lowered, and the hardness of the material for forming the first length region a1 and the hardness of the material for forming the second length region a2 are made different from each other, so that a material having lower hardness is selected for the material of the second length region a2 than the material of the first length region a1. In other words, the hardness of the distal region A of the tubular main body 10 is made low with the joining line D further on the distal side than the change portion B as a boundary. The catheter 1 having the tubular main body 10 as described above maintains a higher hardness up to the vicinity of the tip than in the invention disclosed in Japanese Unexamined Patent Application, First Publication No. 2012-196389 shown as PTL 1, and the runnability in a blood vessel or the torque properties of the tubular main body 10 is improved, so that it is possible to prevent the occurrence of kink. Further, in this embodiment, the hardness of the tip T is reduced, so that the contact of the tip T of the tubular main body 10 with the body cavity can be softened, and therefore, damage, perforation, or spasm of the blood vessel can be prevented.

Further, in this embodiment, the resin material is switched in the distal region A. In contrast, in the invention disclosed in PTL 1, the resin material is switched at the displacement portion (tapered portion). The work of switching the resin material at a portion where the thickness continuously changes is higher in difficulty level than the work of switching the resin material at a portion where the thickness is constant, and variation in the position of the switching easily occurs. Therefore, this embodiment can further enhance the reproducibility of the switching position of the resin material of the tubular main body 10 than in the invention disclosed in PTL 1.

### (Method of Manufacturing Catheter)

Next, a method of manufacturing the catheter 1 shown in FIGS. 6(a) and 6(b) will be briefly described. First, the tubular main body 10 of the catheter 1 is formed by extruding the inner layer 24 into a mold that matches the shape of the inner layer 24. Next, the wire reinforcement layer 30 is formed by winding the reinforcement wire 32 around the inner layer 24. The winding of the reinforcement wire 32 is performed by a dedicated device such that the winding pitch of the reinforcement wire 32 becomes constant. Next, in this embodiment, a core material is created by disposing a plurality of sub-tubes 40 so as to be in contact with the outer surface of the wire reinforcement layer 30 and winding the holding wire 70. A plurality of resin tubes corresponding to the proximal region C, the change portion B, the first length region a1, and the second length region a2 are arranged in this order from the proximal side on the core material, thereby covering the core material. The resins corresponding to the proximal region C, the change portion B, the first length region a1, and the second length region a2 show that the respective resins correspond to the resin hardness and lengths of the proximal region C, the change portion B, the first length region a1 and the second length region a2.

The tubes that form the outer layers 50 in the proximal region C, the change portion B, the first length region a1, and the second length region a2 are prepared in advance by extrusion molding or the like, and are processed into lengths corresponding to the proximal region C, the change portion B, the first length region a1, and the second length region a2. At this time, in this embodiment, in order to obtain the curved joining line D shown in FIG. 6, concavities and convexities are provided on the surfaces adjacent to each other and facing each other, of the resin tube forming the first length region a1 and the resin tube forming the second length region a2, and these resin tubes are arranged on the core material such that the concavities and convexities are fitted to each other, thereby covering the core material.

Next, the inner layer 24, the wire reinforcement layer 30, the sub-tube 40, and the resin tubes are covered with a heat-shrinkable material and heated. The heating temperature is set to a temperature higher than the softening point temperature of the resin having the highest softening point temperature, among the resins configuring the outer layers 50 (the proximal region C, the change portion B, the first length region a1, and the second length region a2).

By the above processes, the proximal region C, the change portion B, the first length region a1, and the second length region a2 are melted and integrated with each other at each boundary, whereby the outer layer 50 is formed. Thereafter, various processes such as forming a hydrophilic layer (not shown) on the surface of the outer layer 50 and inserting the operating line 60 into the sub-tube 40 are performed.

### [Modification Example]

Next, modification examples of this embodiment will be described.

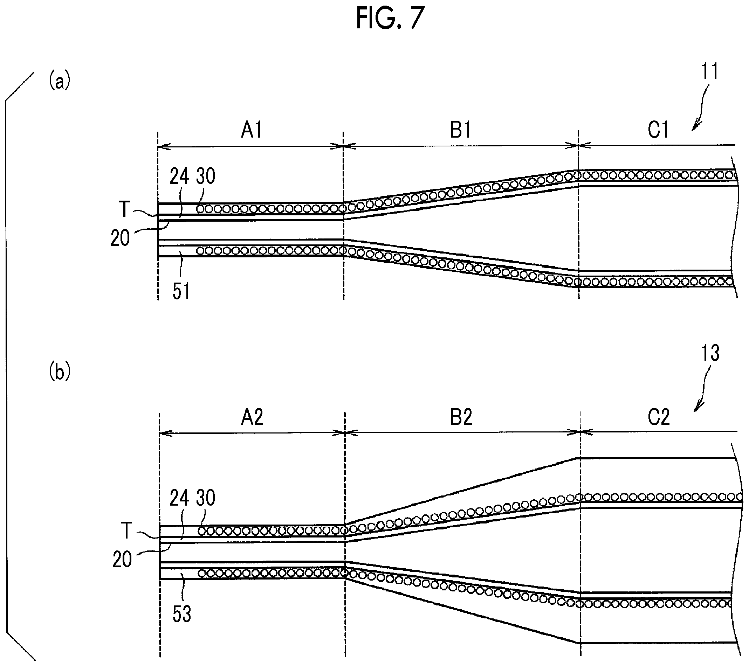

FIGS. 7(a) and 7(b) are diagrams describing the modification examples of this embodiment. FIG. 7(a) is a longitudinal sectional view of a tubular main body 11 which is a modification example of the tubular main body 10. The tubular main body 11 shown in FIG. 7(a) has a tapered outer layer 51 in which both the outer diameter of the tubular main body 11 and the outer diameter of the main lumen 20 continuously increase from the tip toward the base end. At this time, in a change portion B1 of the tubular main body 11, both the outer diameter of the tubular main body 11 and the outer diameter of the main lumen 20 increase with the same inclination (the wall thickness does not change) from the tip toward the base end.

FIG. 7(b) is a longitudinal sectional view of a tubular main body 13 which is a modification example of the tubular main body 10. The tubular main body 13 shown in FIG. 7(b) has a tapered outer layer 53 in which both the outer diameter of the tubular main body 13 and the outer diameter of the main lumen 20 continuously increase from the tip toward the base end. However, in a change portion B2 of the tubular main body 13, the outer diameter of the tubular main body 13 increases with a larger inclination than in the outer diameter of the main lumen 20 (the wall thickness becomes thick) from the tip toward the base end of the tapered outer layer 53.

Further, the catheter of this embodiment is not limited to the configuration described above. For example, various constituent elements of the catheter 1 of this embodiment do not need to be individually independent. For example, a plurality of constituent elements may be formed as one member, one constituent element may be formed of a plurality of members, a certain constituent element may be a part of another constituent element, and a part of a certain constituent element may overlap a part of another constituent element.

The first embodiment includes the following technical ideas.
(1) A catheter including: a tubular main body made of resin and having a change portion in which rigidity resulting from a tubular form continuously decreases from the base end side toward the tip side in an axial direction, wherein a first length region and a second length region located closer to the distal side than the first length region are defined in the tubular main body further on the distal side than the change portion, and a resin hardness of the second length region is lower than a resin hardness of the first length region.
(2) The catheter according to the above (1), wherein the change portion has a uniform resin hardness.
(3) The catheter according to the above (1) or (2), wherein a joining line between the first length region and the second length region is non-uniform in distance from a plane perpendicular to the axial direction of the tubular main body.
(4) The catheter according to any one of the above (1) to (3), wherein the tubular form is at least one element of a wall thickness, an outer diameter, and an inner diameter of the tubular main body.

### [Second Embodiment]

### (Catheter)

FIG. 1 is a diagram describing a catheter 1 of a second embodiment of the present invention. FIG. 2 is a cross-sectional view of the catheter 1 taken along arrow II-II shown in FIG. 1. FIG. 3 is a longitudinal sectional view of the catheter 1 taken along arrow III-III shown in FIG. 2. The catheter 1 of this embodiment is an active catheter in which the operating line 60 is disposed in the interior of the tubular main body 10 and the tip T of the tubular main body 10 is bent by operating the operation part 90 and the bending operation part 92. However, the catheter according to the present invention is not limited thereto and may be an inactive catheter which is not provided with the operating line 60 or the operation part 90.

The catheter 1 of this embodiment has the operation part 90 provided at the base end of the tubular main body 10. Further, the end portion on the side opposite to the side where the tubular main body 10 is connected to the operation part 90 is defined as the tip T. The operation part 90 configures an operation mechanism for performing a bending operation of the distal portion DE of the tubular main body 10 together with the operating line 60 (refer to FIGS. 2 and 3). The operation part 90 of this embodiment has the main body case 94 which is gripped by an operator's hand, and the bending operation part 92 provided to be rotatable with respect to the main body case 94. The base end of the tubular main body 10 is introduced into the interior of the main body case 94. The recessed portion 95 is formed in the main body case 94 at a position which is in contact with the bending operation part 92. The slider 98 which slides to be able to advance toward and retreat from the bending operation part 92 is provided in the recessed portion 95.

The operation part 90 is provided with the hub 96 provided in communication with the main lumen 20 of the tubular main body 10. A syringe (not shown) is mounted to the hub 96. The hub 96 is provided at a rear end portion of the main body case 94, and the syringe is mounted from the rear of the hub 96. A drug solution or the like is injected into the hub 96 by the syringe, whereby the drug solution or the like can be supplied into the body cavity of a subject through the main lumen 20 (FIGS. 2 and 3) which is a hollow portion of the tubular main body 10. As the drug solution or the like, in addition to a drug solution such as a contrast agent, a liquid anticancer agent, a physiological saline, or NBCA (n-butyl-2-cianoacrylate) which is used as an instant adhesive, a medical device such as a detachable coil or beads (embolus globular substances) can be given as an example.

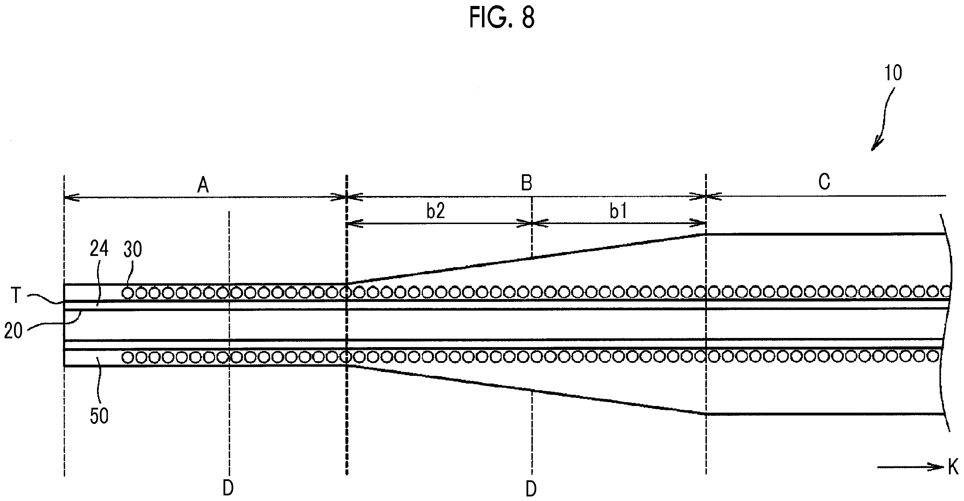

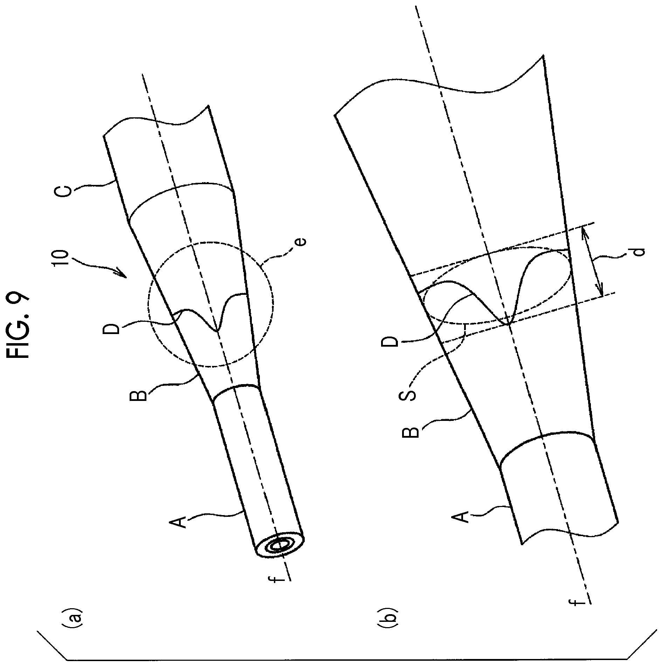

Further, in this embodiment, a first length region b1 (refer to FIG. 8) and a second length region b2 (refer to FIG. 8) adjacent to the first length region and located closer to the distal side than the first length region are defined in the tubular main body 10. Then, the resin hardness of the second length region is lower than the resin hardness of the first length region, and the joining line D (refer to FIG. 9) between the first length region and the second length region is non-uniform in distance from a plane perpendicular to the axial direction of the tubular main body. That is, in this embodiment, in the tubular main body 10, the first length region having a predetermined length is defined. Then, the second length region adjacent to the first length region is defined further on the distal side than the first length region. The first length region and the second length region may have the same length or may have different lengths. The tubular main body 10 is made of resin (hereinafter also referred to as a "resin material") as a material, and the hardness of the resin material of the second length region is lower than the hardness of the resin material of the first length region. Therefore, the tubular main body 10 of this embodiment has a portion where regions having different resin materials are adjacent to each other. The different resin materials adjacent to each other are partially melted and joined to each other by heating, pressurization, and the like.

According to the above configuration, the joining line D between the first length region b1 and the second length region b2 is non-uniform in distance from a plane perpendicular to the axial direction of the tubular main body, and therefore, the hardness of the material gently changes in a portion around the joining line. For this reason, the above configuration can prevent the occurrence of the kink resulting from the discontinuity of the rigidity (hardness) of the tubular main body 10.

The first length region b1, the second length region b2, and the joining line D will be described in detail later.

The tubular main body 10 shown in FIG. 1 is also called a sheath and is a hollow tubular and long member in which the main lumen 20 is formed in the interior thereof. The tubular main body 10 is a tubular main body made of resin and having a "change portion" in which rigidity resulting from a tubular form continuously decreases from the base end side toward the tip side in the axial direction (the direction of an axis passing through the center point of the cross section of the main lumen 20). Here, the "rigidity resulting from a tubular form" is rigidity resulting from the inner diameter or outer diameter of the tubular main body 10 and the wall thickness of the tubular main body 10. That is, the "tubular form" includes at least one of the wall thickness, the outer diameter, and the inner diameter of the tubular main body as an element. The rigidity resulting from the form of the tubular main body 10 becomes small if the inner diameter or the outer diameter of the tubular main body 10 is relatively small, and becomes large if the inner diameter or the outer diameter of the tubular main body 10 is large. Further, the rigidity becomes large if the wall thickness of the tubular main body 10 is relatively thick, and becomes small if the wall thickness of the tubular main body 10 is thin.

In order to change the rigidity resulting from the tubular form of the tubular main body 10 in a "continuous" manner, in an example of this embodiment, the tubular main body 10 has a tapered shape in which the outer diameter of the tubular main body 10 continuously increases (the wall thickness becomes thick) without changing the outer diameter of the main lumen 20 from the tip toward the base end. In this way, the rigidity resulting from the form of the tubular main body 10 continuously increases from the tip T toward the base end.

Here, prior to the description of the first length region and the second length region, an example of the structure of the tubular main body 10 will be described. However, the tubular main body 10 according to the present invention is not limited to the following laminated configuration.

As shown in FIGS. 2 and 3, the tubular main body 10 has a laminated structure. The tubular main body 10 is configured by laminating the inner layer (main tube) 24 and the outer layer 50 in order from the inner diameter side with the main lumen 20 as the center. A hydrophilic layer (not shown) is formed on the outer surface of the outer layer 50. The inner layer 24 and the outer layer 50 are formed of a flexible resin material and each has a circular tube shape and a substantially uniform thickness in the circumferential direction.

The inner layer 24 is the innermost layer of the tubular main body 10, and the main lumen 20 is defined by the inner wall surface thereof. The shape of the cross section of the main lumen 20 is not particularly limited. However, in this embodiment, it is a circular shape. In this specification, the term "cross section of the main lumen" refers to a cross section having a shape defined by the outer peripheral surface of the main lumen 20 (a cylinder long in the axial direction of the main lumen 20). Further, in this embodiment, the diameter of the cross section of the tubular main body 10 is a circular shape, and the diameter of the tubular main body 10 differs depending on the position in a longitudinal direction (the axial direction of the main lumen 20), so that in a part or the entire length region of the tubular main body 10, the tubular main body 10 has a tapered shape in which the diameter of the tubular main body 10 continuously increases from the tip toward the base end.

As the material of the inner layer 24, a fluorine-based thermoplastic polymer resin can be given as an example. As the fluorine-based thermoplastic polymer resin, specifically, polytetrafluoroethylene (PTFE), polyvinylidene fluoride (PVDF), and perfluoroalkoxy fluororesin (PFA) can be given as examples. The inner layer 24 is configured with such a fluorine-based polymer material, whereby the delivery property when supplying the drug solution or the like through the main lumen 20 is improved. Further, the sliding resistance when inserting a guide wire or the like into the main lumen 20 is reduced.

The wire reinforcement layer 30 is a protective layer that is provided further on the inner diameter side than the operating line 60 in the tubular main body 10 to protect the inner layer 24. The wire reinforcement layer 30 is present on the inner diameter side of the operating line 60, whereby it is possible to prevent the operating line 60 from penetrating from the outer layer 50 to the inner layer 24 and being exposed to the main lumen 20.

The wire reinforcement layer 30 is formed by winding the reinforcement wire 32. As the material of the reinforcement wire 32, in addition to a metal material such as tungsten (W), stainless steel (SUS), a nickel titanium alloy, steel, titanium, copper, a titanium alloy, or a copper alloy, a resin material such as polyimide (PI), polyamideimide (PAI), or polyethylene terephthalate (PET), which has higher shear strength than the inner layer 24 and the outer layer 50, can be used. In this embodiment, a fine stainless steel wire is used as the reinforcement wire 32.

The number of the reinforcement wires 32 or the number of meshes is not particularly limited. Here, the number of meshes of the wire reinforcement layer 30 refers to the number of intersections (the number of eyes) per unit length (1 inch) viewed in the extending direction of the reinforcement wire.

The reinforcement wire 32 is wound obliquely around the inner layer 24. An angle formed by the extending direction of the reinforcement wire 32 with respect to the circumferential direction around the inner layer 24 is referred to as a pitch angle of the reinforcement wire 32. In a case where the reinforcement wire 32 is wound at a dense pitch, the pitch angle becomes a small angle. Conversely, in a case where the reinforcement wire 32 is wound at a shallow angle along the axis of the tubular main body 10, the pitch angle becomes a large angle close to 90 degrees. The pitch angle of the reinforcement wire 32 of this embodiment is not particularly limited. However, it can be 30 degrees or more, preferably 45 degrees or more and 75 degrees or less.

The number of the reinforcement wires 32 configuring the wire reinforcement layer 30 is not particularly limited. However, in this embodiment, the wire reinforcement layer 30 formed by 16 reinforcement wires 32 is illustrated.

The sub-tube 40 is a hollow tubular member that defines a sub-lumen. The sub-tube 40 is embedded in the outer layer 50. The sub-tube 40 can be made of, for example, a thermoplastic polymer material. As the thermoplastic polymer material, a low friction resin material such as polytetrafluoroethylene (PTFE), polyetheretherketone (PEEK), or a tetrafluoroethylene/hexafluoropropylene copolymer (FEP) can be given as an example. The sub-tube 40 is made of a material having higher bending rigidity and tensile elastic modulus than the outer layer 50.

Two sub-tubes 40 are disposed on the same circumference so as to surround the main lumen 20. Instead of this embodiment, three or four sub-tubes 40 may be disposed around the main lumen 20 at equal intervals. In this case, the operating lines 60 may be disposed in all the sub-tubes 40, or the operating lines 60 may be disposed in some of the sub-tubes 40. The sub-lumen 42 is formed in the sub-tube 40, and the operating line 60 is movably inserted into the sub-lumen 42.

As described above, the catheter 1 of this embodiment is an active type, and is provided with the sub-tube 40 and the holding wire 70, in addition to the operating line 60 and the operation part 90. However, in a case where the catheter of this embodiment is an inactive type, such a configuration is not necessary.

The operating line 60 is loosely inserted to be slidable with respect to the sub-tube 40. The tip of the operating line 60 is fixed to the distal portion DE of the tubular main body 10. A tensile force is applied to a position eccentric with respect to the axis of the tubular main body 10 by pulling the operating line 60 to the base end side, and therefore, the tubular main body 10 is bent. For this reason, it is possible to cause the catheter 1 to select and enter any blood vessel among the branched blood vessels.

The operating line 60 may be formed of a single wire. However, it may be a stranded wire formed by twisting a plurality of thin wires together. The number of fine wires configuring one stranded wire of the operating wire 60 is not particularly limited but is preferably 3 or more.

As the operating line 60, a metal wire such as low-carbon steel (a piano wire), stainless steel (SUS), a corrosion-resistant coated steel wire, titanium or a titanium alloy, or tungsten can be used. In addition to this, as the operating line 60, polyvinylidene fluoride (PVDF), high-density polyethylene (HDPE), poly(paraphenylenebenzobisoxazole) (PBO), polyetheretherketone (PEEK), polyphenylene sulfide (PPS), polybutylene terephthalate (PBT), polyimide (PI), polytetrafluoroethylene (PTFE), or a polymer fiber such as a boron fiber can be used.

The holding wire 70 is wound in a spiral shape so as to surround the outside of the pair of sub-tubes 40 disposed to face each other around the main lumen 20. The winding shape of the holding wire 70 of this embodiment is an approximately elliptical shape or an approximately rhombic shape in which the direction of the major axis thereof corresponds to the arrangement direction of the sub-tubes 40. In FIG. 3, the holding wire 70 in which the winding shape forms an approximately rhombic shape is shown by a broken line. The holding wire 70 is in contact with the peripheral surfaces of the sub-tubes 40, specifically, the outer surface corresponding to the side opposite to the axis of the main lumen 20. Here, the approximately rhombic shape means that a first diagonal line is longer than a second diagonal line and the first diagonal line and the second diagonal line are substantially orthogonal to each other. The approximately rhombic shape mentioned here includes a flat polygon such as a kite shape, a flat hexagon, or a flat octagon, in addition to a rhombus. Further, the approximately elliptical shape includes an eccentric circle shape such as an ovoid shape, in addition to an elliptical shape or an oval shape.

In this embodiment, an aspect is exemplified in which the shape of the cross section of the main lumen 20 is a circular shape, the main lumen 20 is disposed at the center of the tubular main body 10, and the two sub-tubes 40 are disposed around the main lumen 20 so as to face each other by 180 degrees. However, the shape of the cross section of the main lumen 20 is not limited to a circular shape and may be another shape such as an ellipse or a rectangle. Further, three or more (N) sub-tubes 40 may be equally distributed and disposed around the main lumen 20. In this case, the winding shape of the holding wire 70 may be a corner-rounded N-polygonal shape with each sub-tube 40 as a corner.

As the material of the holding wire 70, any of the above-described metal materials or resin materials that can be used as the reinforcement wire 32 can be used. In this embodiment, the holding wire 70 is formed to include a material of a different type from that of the reinforcement wire 32. The ductility of the holding wire 70 is preferably higher than the ductility of the reinforcement wire 32. Specifically, austenitic soft stainless steel (W1 or W2), which is an annealed material, or copper or a copper alloy is used for the holding wire 70, while tungsten or stainless spring steel can be used for the reinforcement wire 32.

The outer layer 50 is a circular tube-shaped portion that configures the main wall thickness of the tubular main body 10. The wire reinforcement layer 30, the sub-tube 40, and the holding wire 70 are provided in order from the inner diameter side in the interior of the outer layer 50. The wire reinforcement layer 30 and the holding wire 70 are disposed coaxially with the tubular main body 10.

In this embodiment, the resin hardness of the first length region and the resin hardness of the second length region of the tubular main body 10 being different from each other means that the resin hardness of the outer layer 50 configuring the main wall thickness of the tubular main body 10 is different. However, the outer layer 50 may be a single layer or a multilayer, and when the outer layer 50 is a multilayer, it indicates that the resin hardness of the first length region and the resin hardness of the second length region are different from each other with respect to the thickest layer of a plurality of layers.

As the material of the outer layer 50, a thermoplastic polymer material can be used. As the thermoplastic polymer material, polyimide (PI), polyamideimide (PAI), polyethylene terephthalate (PET), polyethylene (PE), polyamide (PA), polyamide elastomer (PAE), nylon elastomer such as polyether block amide (PEBA), polyurethane (PU), ethylene-vinyl acetate resin (EVA), polyvinyl chloride (PVC), or polypropylene (PP) can be given as an example. An inorganic filler may be mixed with the outer layer 50. As the inorganic filler, a contrast agent such as barium sulfate or bismuth subcarbonate can be exemplified. The contrast agent is mixed with the outer layer 50, whereby the X-ray contrast property of the tubular main body 10 in the body cavity can be improved.

In a case of switching the resin hardness by making the resin material of the first length region and the resin material of the second length region different from each other, it is preferable in terms of adhesion that the resin material of the first length region and the resin material of the second length region are the same type of resin materials having different hardness. The same type of resin materials having different hardness refer to, for example, resin materials in which there is a commonality in resin structure and the degree of cross-link is different, or the hardness is different resulting from a different component or addition amount of a filler or an elastomer, or the like.

Next, the change portion, the first length region, and the second length region of this embodiment described above will be described.

FIG. 8 is a schematic longitudinal sectional view showing the distal portion DE of the tubular main body 10 in an enlarged manner. The illustrated distal portion DE has the tapered change portion B in which the outer diameter of the tubular main body 10 continuously decreases from the base end side toward the tip side, the proximal region C located closer to the proximal side than the change portion B. and the distal region A located closer to the distal side than the change portion B. The inner circumference of the main lumen 20 is constant even in the change portion B. The inclination of the outer diameter of the tubular main body 10 may be the same throughout the change portion B, or the inclination may change in the middle. The outer diameter of the main lumen 20 may be constant even in the change portion B, or may have the same inclination throughout the change portion B, or the inclination may change in the middle.

The change portion B is a portion in which the rigidity resulting from the form in the direction of the axis f continuously decreases from the base end K side toward the tip T side, in the tubular main body 10. In this embodiment, the joining line D between the first length region b1 and the second length region b2 is formed in the change portion B. The joining line D is not limited to being provided in the change portion B and may be provided at the distal region A, the boundary between the distal region A and the change portion B, the boundary between the change portion B and the proximal region C, and any position on the proximal region C.

From the viewpoint of a balance that has excellent blood vessel selectivity or guide wire followability in a blood vessel branch while providing good runnability in a blood vessel or torque properties of the catheter, it is preferable that, with regard to the resin hardness of the first length region b1 and the resin hardness of the second length region b2, the value of (Shore hardness of the first length region b1)/(Shore hardness of the second length region b2) be set to be 1.1 or more and 2.0 or less. Further, from the viewpoint of a balance that has excellent blood vessel selectivity or guide wire followability in a blood vessel branch while providing good runnability in a blood vessel or torque properties of the further catheter, it is preferable that the value of (Shore hardness of the first length region b1)/(Shore hardness of the distal region A) be set to be 1.1 or more and 2.0 or less.

Among these, in the example shown in FIG. 8, nylon elastomer is used for the outer layers 50 in the distal region A, the change portion B, and the proximal region C, and the length, the diameter φ of the main lumen 20 (an index of the area of the cross section), and the Shore hardness of the outer layer 50 can be set as follows, for example.
Distal region A: length 15 mm, φ0.8 mm, Shore hardness 25D
Change portion B: length 10 mm, φ0.8 mm to 0.9 mm, Shore hardness 35D (the first length region b1, length 5 mm), Shore hardness 25D (the second length region b2, length 5 mm)
Proximal region C: length 10 mm, φ0.9 mm, Shore hardness 35D

That is, in the above configuration, the resin hardness of the outer layer 50 that configures each of the first length region b1 and the proximal region C is uniform.

However, the size or the resin hardness of each of the distal region A, the change portion B, and the proximal region C is not limited to the above numerical values or ratios as a matter of course. The size and resin hardness of each part are determined according to the use or the like of the catheter 1.

FIGS. 9(a) and 9(b) are perspective views showing the portion of the change portion B in the tubular main body 10 and are diagrams showing the joining line D in the change portion B. FIG. 9(a) shows the entire change portion B, and FIG. 9(b) is an enlarged diagram of the portion indicated by a broken line e in FIG. 9(a). The distance d of the joining line D on the change portion B from a plane (the cross section S of the tubular main body 10) perpendicular to the direction of the axis f of the tubular main body 10 is non-uniform. Here, the distance d from the cross section S refers to the distance d between a point on the cross section S and the intersection of a line extending parallel to the axis f from this point and the joining line D. As shown in FIG. 9(b), the distance d varies depending on the position on the cross section S.

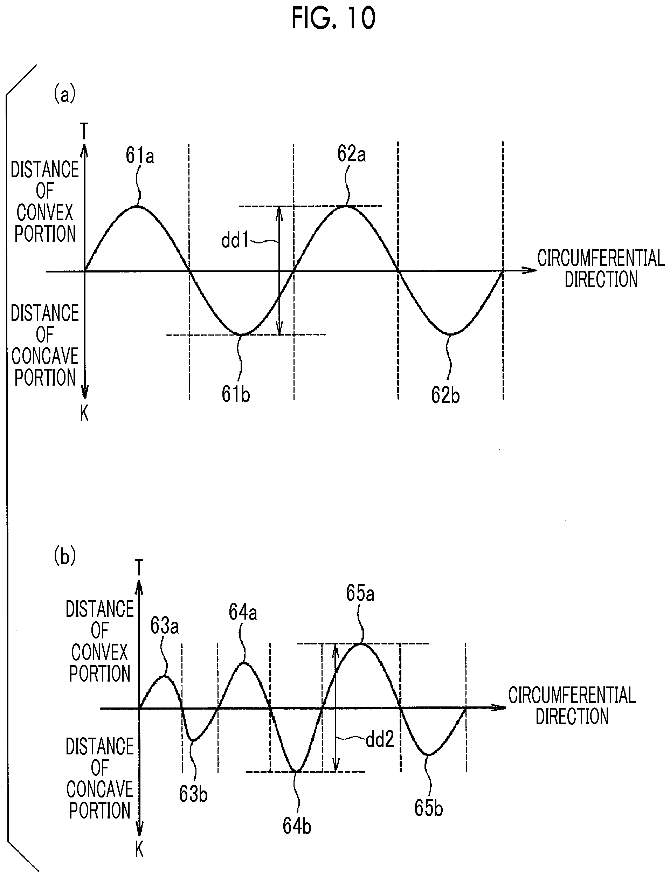

FIGS. 10(a) and 10(b) are diagrams describing the joining line D. In both FIG. 10(a) and FIG. 10(b), the vertical axis indicates the distance from the cross section S to the joining line D (including the distance to an apex of a convex portion and an apex of a concave portion). The horizontal axis indicates the length in the circumferential direction of the cross section S passing through one point on the joining line D continuous in the circumferential direction of the tubular main body 10. FIG. 10(a) shows one example of the joining line D in which the combination of a convex portion and a concave portion is at least one set in the circumferential direction of the tubular main body 10. In the example shown in FIG. 10(a), there are two combinations; a combination of a convex portion 61a and a concave portion 61b and a combination of a convex portion 62a and a concave portion 62b, and the shape in which the convex portion 61a and the concave portion 61b are arranged in series and the shape in which the convex portion 62a and the concave portion 62b are arranged in series are substantially equal to each other. However, this embodiment is not limited to the example in which the shapes are the same, and the shapes may be different from each other. Further, in this embodiment, since it is sufficient if the combination of the convex portion and the concave portion is at least one or more, there is no limitation to two combinations, and the combination may be one or any number of two or more.

Further, FIG. 10(b) shows one example in which the combinations of convex portions and concave portions are at least two sets in the circumferential direction of the tubular main body 10. In the example shown in FIG. 10(b), there are three combinations; a combination of a convex portion 63a and a concave portion 63b, a combination of a convex portion 64a and a concave portion 64b, and a combination of a convex portion 65a and a concave portion 65b, and the shape in which the convex portion 63a and the concave portion 63b are arranged in series, the shape in which the convex portion 64a and the concave portion 64b, and the shape in which the convex portion 65a and the concave portion 65b are arranged in series are difference from each other. Here, the shape in which the convex portion and the concave portion are arranged in series refers to a series of curves which includes the convex portion and the concave portion. Further, in this embodiment, since it is sufficient if the combinations of the convex portions and the concave portions are at least two or more, there is no limitation to three combinations, and the combinations may be two or any number of two or more.

As will be described later, in this embodiment, in order to form the joining line D having a concavity and a convexity, as one example, concavities and convexities are formed in advance on the facing surfaces of the member forming the first length region b1 and the member forming the second length region b2. In a case where the shapes of the concave portion and the convex portion can be sufficiently controlled by the concavity and convexity formed in advance, the shapes of the concave portion and the convex portion of the joining line D can be made substantially the same.

As described above, it is sufficient if the joining line D is a curve surrounding the circumference of the change portion B of the tubular main body 10, and the cycles of the concavity and the convexity or the amplitudes of the concavity and the convexity may be constant, or may be random.

Further, in this embodiment, the first length region b1 and the second length region b2 are joined to each other with the intention that the joining line D becomes a curve. For this reason, the joining line D has a waveform having a larger amplitude than a joining line that unintentionally draws a curve in a joining process. In the example shown in FIG. 10(a), a difference in the distance in the axial direction between the most distal side (shown with the tip T) and the most base end K side of the joining line D is shown as a length dd1. Further, in the example shown in FIG. 10(b), a difference in the distance in the axial direction between the most distal side and the most base end K side of the joining line D is shown as a length dd2.

As described above, the tubular main body 10 has a laminated structure which includes at least the inner layer 24 and the outer layer 50. In this embodiment, both the length dd1 and the length dd2 are set to be equal to or larger than the wall thickness of the outer layer 50 at the position based on the joining line D on the tubular main body 10 (double the wall thickness), and equal to or less than three times the outer diameter of the tubular main body 10. In this way, resins having different hardness sufficiently enter toward each other between the first length region b1 and the second length region b2, so that it is possible to make a change in hardness between the first length region b1 and the second length region b2 moderate.

The "position based on the joining line D" in this embodiment may be, for example, the position of a cross section S_{1/2} of the tubular main body 10 including a point that halves each of the lengths dd1 and dd2 of the joining line D. At this time, the wall thickness of the outer layer becomes the thickness of the outer layer 50 of the tubular main body 10 at a position intersecting the cross section S_{1/2}.

Further, in this embodiment, each of the lengths dd1 and dd2 may be set to be, for example, equal to or larger than the radius of the cross section S or equal to or larger than the radius and equal to or less than three times the radius.

In this embodiment, as described above, the concave portion or the convex portion of the joining line D has a shape that protrudes toward the tip T side or the base end side. In this way, the hardness gently changes in the joining line D, and thus, when the catheter 1 is bent, the region ranging from the change portion B to the distal region A is smoothly curved, so that it is possible to prevent cracks or damages (kink) from occurring in the change portion B. Further, by forming such a joining line in the change portion B, a change in hardness becomes more moderate in the change portion B. For this reason, even if slight variation occurs in the position of the joining line D, it is possible to absorb variation in the function such as the runnability in a blood vessel, the runnability in a blood vessel or the torque properties for each product of the catheter 1.

### (Method of Manufacturing Catheter)

Next, a method of manufacturing the catheter 1 shown in FIGS. 9(a) and 9(b) will be briefly described. First, the tubular main body 10 of the catheter 1 is formed by extruding the inner layer 24 into a mold that matches the shape of the inner layer 24. Next, the wire reinforcement layer 30 is formed by winding the reinforcement wire 32 around the inner layer 24. The winding of the reinforcement wire 32 is performed by a dedicated device such that the winding pitch becomes constant. Next, in this embodiment, a core material is created by disposing a plurality of sub-tubes 40 so as to be in contact with the outer surface of the wire reinforcement layer 30 and winding the holding wire 70. A plurality of resin tubes corresponding to the proximal region C, the first length region b1, the second length region b2, and the distal region A are arranged in this order from the proximal side on the core material, thereby covering the core material. The resins corresponding to the proximal region C, the first length region b1, the second length region b2, and the distal region A show that the respective resins correspond to the resin hardness and lengths of the proximal region C, the change portion B, the first length region b1, the second length region b2, and the distal region A.

The tubes that form the outer layers 50 in the proximal region C, the first length region b1, the second length region b2, and the distal region A are prepared in advance by extrusion molding or the like, and are processed into lengths corresponding to the proximal region C, the first length region b1, the second length region b2, and the distal region A. At this time, in this embodiment, in order to obtain the curved joining line D shown in FIG. 9, concavities and convexities are provided on the surfaces adjacent to each other and facing each other, of the resin tube forming the first length region b1 and the resin tube forming the second length region b2, and these resin tubes are arranged on the core material such that the concavities and the convexities are fitted to each other, thereby covering the core material.

Next, the inner layer 24, the wire reinforcement layer 30, the sub-tube 40, and the resin tubes are covered with a heat-shrinkable material and heated. The heating temperature is set to a temperature higher than the softening point temperature of the resin having the highest softening point temperature, among the resins configuring the outer layers 50 (the proximal region C, the first length region b1, the second length region b2, and the distal region A).

By the above processes, the proximal region C, the first length region b1, the second length region b2, and the distal region A are melted and integrated with each other at each boundary, whereby the outer layer 50 is formed. Thereafter, various processes such as forming a hydrophilic layer (not shown) on the surface of the outer layer 50 and inserting the operating line 60 into the sub-tube 40 are performed.

### [Modification Example]

Next, modification examples of this embodiment will be described.

FIGS. 7(a) and 7(b) are diagrams describing the modification examples of this embodiment. FIG. 7(a) is a longitudinal sectional view of the tubular main body 11 which is a modification example of the tubular main body 10. The tubular main body 11 shown in FIG. 7(a) has a tapered outer layer 51 in which both the outer diameter of the tubular main body 11 and the outer diameter of the main lumen 20 continuously increase from the tip toward the base end. At this time, in the change portion B1 of the tubular main body 11, both the outer diameter of the tubular main body 11 and the outer diameter of the main lumen 20 increase with the same inclination (the wall thickness does not change) from the tip toward the base end. Further, the wall thicknesses of a distal region A1 and a proximal region C1 are constant.

FIG. 7(b) is a longitudinal sectional view of the tubular main body 13 which is a modification example of the tubular main body 10. The tubular main body 13 shown in FIG. 7(b) has a tapered outer layer 53 in which both the outer diameter of the tubular main body 13 and the outer diameter of the main lumen 20 continuously increase from the tip toward the base end. However, in the change portion B2 of the tubular main body 13, the outer diameter of the tubular main body 13 increases with a larger inclination than in the outer diameter of the main lumen 20 (the wall thickness becomes thick) from the tip toward the base end of the outer layer 53. Further, in a distal region A2 and a proximal region C2, although the proximal region C2 is thicker than the distal region A2, the wall thickness of each of the distal region A2 and the proximal region C2 is constant.

Further, the catheter of this embodiment is not limited to the configuration described above. For example, various constituent elements of the catheter 1 of this embodiment do not need to be individually independent. For example, a plurality of constituent elements may be formed as one member, one constituent element may be formed of a plurality of members, a certain constituent element may be a part of another constituent element, and a part of a certain constituent element may overlap a part of another constituent element.

The second embodiment includes the following technical ideas.
(1) A catheter, including: a tubular main body, wherein a first length region and a second length region adjacent to the first length region and located closer to the distal side than the first length region are defined in the tubular main body, a resin hardness of the second length region is lower than a resin hardness of the first length region, and a joining line between the first length region and the second length region is non-uniform in distance from a plane perpendicular to an axial direction of the tubular main body.
(2) The catheter according to the above (1), wherein the joining line has at least one combination of a concave portion and a convex portion in a circumferential direction of the tubular main body.
(3) The catheter according to the above (1), wherein the joining line has at least two combinations of a concave portion and a convex portion in a circumferential direction of the tubular main body, and shapes of a set of the convex portion and the concave portion of the respective combinations are different from each other.
(4) The catheter according to any one of the above (1) to (3), wherein the tubular main body has a laminated structure which includes at least an inner layer and an outer layer, and a difference in distance in the axial direction between a most distal side and a most base end side of the joining line is equal to or larger than a wall thickness of the outer layer at a position based on the joining line of the tubular main body, and equal to or less than three times an outer diameter of the tubular main body.
(5) The catheter according to any one of the above (1) to (4), wherein the tubular main body has a change portion in which rigidity resulting from a tubular form continuously decreases from the base end side toward the tip side in the axial direction.
(6) The catheter according to any one of the above (1) to (5), wherein the joining line between the first length region and the second length region is formed in the change portion.
(7) The catheter according to the above (5), wherein the tubular form is at least one element of a wall thickness, an outer diameter, and an inner diameter of the tubular main body.

### Reference Signs List

1: catheter
10, 11, 13: tubular main body
20: main lumen
24: inner layer
30: wire reinforcement layer
32: reinforcement wire
40: sub-tube
42: sub-lumen
50, 51, 53: outer layer
60: operating line
70: holding wire
90: operation part
92: bending operation part
94: main body case
95: recessed portion
96: hub
98: slider
A, A1, A2: distal region
B, B1, B2: change portion
C, C1, C2: proximal region
D, D1, D2, D3, D4: joining line
DE: distal portion
K: base end
S: cross section
T: tip
al: first length region
a2: second length region
b1: first length region
b2: second length region
d: distance of joining line from cross section
f: axis of tubular main body

## Claims

1. A catheter, comprising:
a tubular main body,
wherein a first length region and a second length region adjacent to the first length region and located closer to a distal side than the first length region are defined in the tubular main body,
a resin hardness of the second length region is lower than a resin hardness of the first length region, and
a joining line between the first length region and the second length region is non-uniform in distance from a plane perpendicular to an axial direction of the tubular main body.

2. The catheter according to Claim 1, wherein the joining line has at least one combination of a concave portion and a convex portion in a circumferential direction of the tubular main body.

3. The catheter according to Claim 1, wherein the joining line has at least two combinations of a concave portion and a convex portion in a circumferential direction of the tubular main body, and shapes of a set of the convex portion and the concave portion of the respective combinations are different from each other.

4. The catheter according to any one of Claims 1 to 3, wherein the tubular main body has a laminated structure which includes at least an inner layer and an outer layer, and a difference in distance in the axial direction between a most distal side and a most base end side of the joining line is equal to or larger than a wall thickness of the outer layer at a position based on the joining line of the tubular main body, and equal to or less than three times an outer diameter of the tubular main body.

5. The catheter according to any one of Claims 1 to 4, wherein the tubular main body has a change portion in which rigidity resulting from a tubular form continuously decreases from a base end side toward a tip side in the axial direction.

6. The catheter according to any one of Claims 1 to 5, wherein the joining line between the first length region and the second length region is formed in the change portion.

7. The catheter according to Claim 5, wherein the tubular form is at least one element of a wall thickness, an outer diameter, and an inner diameter of the tubular main body.
